# EUROPEAN PATENT APPLICATION

(11) **EP 4 778 563 A1**
(43) Date of publication of application: **22.07.2026**
(21) Application number: 25305071.0
(22) Date of filing: 21.01.2025
(51) Int. Cl.: A61M 39/14, A61M 39/18, A61M 39/08, B29C 65/00

(54) **METHOD OF CUTTING AND REARRANGING TWO COLLAPSIBLE TUBES FOR BIOPHARMACEUTICAL USE, DEVICE FOR IMPLEMENTATION OF THE METHOD, USING PINCHING SLEEVES CUT AND REARRANGED TO ENCAPSULATE A BONDING LAYER AROUND THE RECONNECTED TUBE SECTIONS**

(71) Applicant: SARTORIUS STEDIM FMT, 13400 Aubagne (FR)
(72) Inventor: Loiselet, Yanis, 13400 AUBAGNE (FR)
(74) Representative: Plasseraud IP

(57) **Abstract**

A kit of two sleeves (3) is provided, each comprising sleeve parts (3a, 3b) including pins (14). A molding space is formed along sleeve inner face, between ends (3c, 3d) that can delimit such space. The kit is used in a method of joining a first and a second fluid conveying tubes, while also joining a third and a fourth fluid conveying tubes, by providing two tube pieces that are wrapped locally, each by a sleeve of the kit, before being pinched and cut together with the two sleeves, typically with a cutting member separating two tube holders involved in the pinching operation. Thanks to the pins (14), the molding space remains available for a curable material and the joining is performed after permutating two cut tube sections to provide sleeve-protected alignment of the tubes by proper pair, by a peripheral curing step using said material.

## Description

### FIELD OF THE INVENTION

The present disclosure relates generally to systems having tubes, and more particularly, a tube disconnection and connection system and a method for crimping a tube section of a tube and allowing a cut for separating two tube members.

### BACKGROUND OF THE INVENTION

In the pharmaceutical, chemical, and other fields that use tubing in the processing and transport for fluids (such as biopharmaceutical fluids), there is often a need for separating two tube members of the tubing, by applying a sealing action and cutting, and the rearranging the tube members in a modified circuit. In the field of biotechnology for instance, there is a need for bioprocesses that allow flexibility, with changes in the connections to the disposable containers or bioreactor parts. New processes and procedures are being developed for closed system processes. Typically, many of these procedures require connecting and disconnecting a variety of different bioprocess containers to and from the cell culture or content of a bioreactor.

In biopharmaceutical production, drugs are produced and stored in single use bags. After production, bags or transfer lines also need to be disconnected to allow a transfer process. Use of connectors should be avoided as manipulation can cause risks. Furthermore, there is no flexibility for choosing the location for disconnecting and rearranging tube parts when connectors are involved.

In the past, more and more solutions were directed to welding so that the tubes are welded together instead of using plastic connectors for fluid connection. Unfortunately, a significant number of welding errors is encountered. In addition to posing an environmental contamination risk, welding errors can result in open connections that can compromise the growth of the entire cell culture.

A known method of connecting two silicone hose sections at cut end faces (of annular shape) is known from document WO 2023138952 A1, using a cutting unit for preliminary cutting two distinct hoses at respective cutting sites, with a mechanical cut performed at a temperature less than 50°C, before placing the two hose sections in a specific mold and then molding an annular outer layer inside this mold with a sterilizing operation, to form an annular junction around the two silicone hose sections. A new connection is thus obtained. This method is particularly prone to errors. Operations of emptying and cutting the tubes are performed independently from the subsequent steps and no control of sterility is ensured once the tube hose sections are obtained and displaced. This creates a complexity in the way the process must be performed (many requirements to keep sterility), at least until starting the molding step.

Other connections can be obtained after a tube disconnection, for instance as in document US 4832773 A, using a common technique of welding the tubes together. This document discloses a welding utilizing a heated wafer which cuts through a pair of tubes either simultaneously or sequentially. The heated tube ends are in a molten condition so that by shifting the tubes a molten tube end from one of the tubes is disposed in line with a molten tube end from the other tube. The molten ends are pressed together to weld the two tube sections and form a unitary tube. Such a welding method is dedicated to thermoplastic polymer tubes but is not suitable for silicone or similar material (non-weldable) used in bioprocesses. Of course, the welding site must be clear and free of any fluid or fluid ingress before a welding process can begin.

There is still room for improving operations that relate to such tubes, especially by cutting them into sections that are closed permanently, while keeping a tube end aseptic, i.e., with their bacterial integrity not challenged, and reconnecting at least one of the tube cut ends with another one tube cut end.

### OBJECTS AND SUMMARY OF THE INVENTION

For improving situation, embodiments of the invention provide an encapsulated connection kit provided with two protecting sleeves/sealing sleeves for surrounding two flexible tubes (of a biopharmaceutical fluid pathway/assembly or circuit) and possibly allowing a rearrangement (rearrangement typically with a pair of tube connections involving four cut end faces obtained from the two flexible tubes made of plastic or silicone, after a cut - preferably simultaneous cut - of the two flexible tubes at tube sections thereof), wherein the kit comprises a first sleeve and a second sleeve forming the two sleeves/sealing sleeves, which are each of resiliently deformable plastic material, each of the first sleeve and the second sleeve being a sleeve comprising:
- a central axis;
- an interior molding space delimited by an inner face (sleeve inner face);
- at least one linking area that allows a full or partial disconnection, and reconnection, of two sleeve parts of the sleeve that provide two interior subspaces for molding, so that a side opening is available for tube insertion between the two subspaces;
- a plurality of pins or protrusions that project radially inward from the inner face, with the plurality of pins or protrusion being distributed circumferentially around the central axis (the pins or protrusions being typically integrally formed with two respective sleeve parts of the sleeve);
wherein each of the first sleeve and the second sleeve is a radially collapsible sleeve that is:
- configured to be pinched at two opposite sides to close a lumen of the corresponding flexible tube passing therethrough, under radial pushing effect of the plurality of pins,
- provided with the inner face, in undeformed configuration, which has a generally circular section so that each of the two sleeves has an inner radius, as measured between the inner face and the central axis in a undeformed sleeve configuration,
wherein the pins or protrusions can be shorter than the inner radius, preferably shorter than half the inner radius.

Thanks to this kit, molding cavities can be provided and follow the rearrangement, so that an additional fixing material can be added around the tube, along the inner face portions of the sleeves. It is understood the sleeves can be used as a protection forming a small molding device, while having the pins or protrusions protruding in the molding space to allow the proper tube compression (squeezing) which is of interest to remove liquid/fluid that can be initially present in the tube(s) at a location for the cut.
The kit of two sleeves, each provided with sleeve parts including the pins/protrusions, allows forming modular molding spaces that are following movement of the tube portions, while being typically remaining delimited between ends of the sleeve. The kit can be used in a method of joining a first and a second fluid conveying tubes, while also joining a third and a fourth fluid conveying tubes, by providing two tube pieces that are wrapped locally, each by a sleeve of the kit, before being pinched and cut together with the two sleeves, typically with a cutting member separating two tube holders involved in the pinching operation. Thanks to the pins or protrusions, the molding space remains available for a curable material and the joining is performed after permutating two cut tube sections to provide sleeve-protected alignment of the tubes by proper pair, by a peripheral curing step using the curable material.

The rearrangement can be performed in a way similar to known techniques for creating two tube connections, using a cutting member or blade acting as a temporary closure member in contact with each of the cut end faces of the tube segments that have just been cut. But in this case, the sleeves are also involved, and sleeve-tube contact can be kept to have the tube segments that follow movement of the associated sleeve wrapping portions associated to these tube segments.

The pins or protrusions are configured to contact the tube and maintain a radial space between the tube and the inner face, so that each interior molding space can extend around the tube and a curable material can coat the pins or protrusions and encapsulate each junction area where two of the cut end faces are axially joined (i.e. with the curable material extending underneath each protecting sleeve surface of said inner face). It is understood that the pins/protrusions can be tube contacting pins maintaining a radial space between the tube and the inner surface, even in compressed state to close the lumen. Compression of the sleeve is allowed by the resiliently deformable material, possibly combined with/enhanced by providing areas of reduced thickness or grooves that provide a design with bellows or longitudinal thinned parts providing each a (small) hinge effect.
The clamping (squeezed) configuration can involve a holder assembly with a pair of housings sized and shaped to receive the sleeves, possibly with abutting walls or parts. In this configuration, the lumen is closed: for the two tubes equipped with the kit, this prevents fluid circulation through the tube section of the first tube in a pinched/squeezed state of this tube section and prevents fluid circulation through the tube region of the second tube in a pinched/squeezed state of this tube region in a pinched state of the tube region. Such configuration with the emptying effect can be advantageously obtained before the cut with each tube already encapsulated, locally, with or without a curable material added to fill the interior molding space in the clamping configuration.

The pair of sleeves can be easily installed at any desired location, with ability to:
- choose flexibly location of the cutting sites (in a suitable area for the respective tubes).
- maintain a molding interior space that extends annular (circumferentially) around a midsection of the tube (before the cut) where a cutting site is present and provided in a transverse plane that is also a cut plane for the corresponding sleeve.
- cover two halves of such interior space when performing a cut of the two sleeves arranged around the corresponding tube, with the cutting member separating these halves.
- provide recombined molding interior spaces by following movement (rotation typically) of two the cut end faces of the tubes parts wrapped and squeezed together with a pair of two sleeve segments extending on a same side relative to the cutting member.
- keep each lumen closed (at and adjacent to the cut end faces obtained from the cut) due to flexibility/radial compressibility of the sleeve material combined with the use of rigid or semi-rigid pins/protrusions less deformable than the tubes that are generally silicon tubes or thermoplastic tubes.

Installation of the kit, by wrapping areas (of similar extension) of the tubes by the two sleeves, can optionally be performed in a clean room. More generally, the sleeve can have any suitable construction, preferably with two parts, that can pass from an open configuration with full or partial disconnection of fixation means carried by the sleeve, to a reconnection configuration with the subspaces (two subspaces when having two sleeve parts) arranged complementary to define the interior molding cavity, with the pins or protrusions preventing the interspace/gap (radial gap between the outer face of the tube and the inner face of the sleeve) forming this cavity to disappear in a squeezed state to close the tube lumen.

In some embodiments of the kit, one or more of the following features are implemented:
- the sleeve has an inside dimension at least as large as the outside diameter of the fluid conveying tube.
- the sleeve is adapted to be installed on the fluid conveying tube at the cutting sites in a substantially coaxial relationship with the fluid conveying tube.
- the sleeve has an external wall with a wall thickness which insulates the fluid conveying tube, locally in an annular region.
- the sleeve consists of a pair of half cylindrical pieces.
- the sleeve is formed of a plastic material, without any metal.
- the interior molding space is shaped and sized so that an encapsulation layer of the curable material can be formed, either inside the sleeve before any cut thereof, or inside a recombined sleeve (with two sleeve parts joined axially at sleeve circular cut ends provided from the two sleeves) obtained after the rearrangement.
- the encapsulation layer extends around a tube reconnection site/junction area where two cut end faces are in axial abutment after the cut and the rearrangement.
- each sleeve can have a total length superior or equal to 20 mm.
- the encapsulation layer can extend at least 10 mm beyond the junction area, respectively in opposite axial directions.
- the pins or protrusions are distributed in the cavity/interior molding space to be provided on at least two opposite sides.
- the pins or protrusions have a length (radial size) superior to 2 mm, preferably superior or equal to 4 or 5 mm.
- the pins or protrusions may be longer than wide.
- all or part of the pins or protrusions are possibly provided with a free end that defines a surface as wide as a diameter taken at half the length of the pin/protrusion.
- each sleeve can have a thickness greater at two opposite axial sleeve ends, as compared to an average thickness in a recessed intermediate area, where the pins and protrusions are provided.
- the sleeve parts, interlocked to obtain the sleeve, can each have a U-shaped profile as viewed in a cross-section (perpendicular to the central axis).

The kit of two sealing/protecting sleeves allows connecting biopharmaceutical flexible tube ends of two tubes in an encapsulated configuration using the two sleeves, according to any suitable cutting and connection method that allows closing the cut end faces of the two respective tubes by the cutting means involved to obtain the four cut end faces.
When using an apparatus similar to the Biowelder^{®} from Sartorius, with sleeve holders instead of tube holders, the cut and rearrangement can be performed very fast, with reconnection that is possibly a sterile reconnection. A clamping action can be started and exerted before the cut, knowing that the sleeves are each squeezable along a transverse direction perpendicular to the central axis, to allow the pins mechanically sealing a tube region/closing a tube lumen of the tube that is sandwiched between the oppositely positioned pins/protrusions of the sleeve that are distributed in the two sides.

Of course, any suitable holding assembly can be provided to maintain the sleeves at the time of the cutting. Rearrangement to interchange the tube sections already covered by a sleeve (each tube section being covered from the midsection of a sleeve) can be performed to immediately obtain a rearranged sleeve, before moving away the blade for instance. Then, the curable material (already present or filled after the cut and typically after the cutting blade retrieval) can be heated or treated by suitable irradiation (UV for instance).

The kit can be used with the sleeves being parallel and kept in two spaced housings during the cutting operation involving a first tube and a second tube that have same inner diameter and outer diameter, respectively, each of the two sealing sleeves being made of a resiliently deformable material and being recessed to have an interspace (radial gap) between the tube cutting site and the corresponding annular cutting site provided at the sleeve that surrounds this tube.

In preferred embodiments, the interior molding space (for a sleeve in assembled/annular configuration) is delimited by two opposite axial end parts formed as constrictions that inwardly project from the inner face. When the sleeve is formed of two similar parts or halves, the two opposite axial end parts may be distributed in the two halves. Each half can form half circumference of the sleeve. Each of the axial end parts can provide an annular radial contact part for radial contact with one of the corresponding tubes, with the first axial end part and the second axial end part being two opposite open axial ends, typically delimiting a circular cross section with a slotted structure at these axial end parts.
The axial end parts can each have a plurality of inwardly orientated radial protrusions that have a same radial extension to provide an annular inner edge (with annular contact surface) in a squeezed state.

Optionally, the sleeve is formed of two molded parts. The two opposite axial end parts may act as mold ends (delimiting the interior molding space) that are configured to be separated by the cut and then used again to delimit a new molding interior space when providing the new connection/tube junction with the sleeve cut sections remaining coupled to corresponding tube segments.

Should the curable material be present before a cut, with each tube already coupled to a sealing sleeve, then it is understood the curable material may be present/provided underneath the two sealing sleeves, along each inner face.
Should the curable material be introduced after the cut (and typically after the rearrangement), after each tube has been coupled to a sealing sleeve, then it is understood the curable material may be present/provided underneath two pairs of rearranged sleeve portions (cut portions extracted via the cut and rearrangement operation applied to the tubes already coupled with the protecting sleeves) from the two protecting sealing sleeves. The curable material can be injected in the newly created interior molding spaces, possibly after an extraction of the cutting blade/cutting means with the cut tube sections being in axial contact and the cut sleeves sections in axial contact as well (possibly with an axial pushing exerted on the sleeves). This may be performed using a suitable device for maintaining:
- the clamping configuration before the cut,
- the clamping configuration after the cut with each cut face (cut face of the tube and cut face of the sleeve) in axial contact with the cutting member,
- an axial contact, after the rearrangement, between the rearranged cut faces from the two tubes and the cutting member on the one hand, and between the rearranged cut faces from the two sleeves and the cutting member on the other hand, and
- the direct sleeve-sleeve axial contact and a direct first tube - second tube contact, after a removal of the cutting blade/member.

Each sleeve of the kit can comprise:
- a first axial end and a second axial end that are two opposite open axial ends (of the sleeve), so that the central axis passes through two opposite openings of the two axial ends;
- a continuous recess, of annular shaped for a connected/closed state of the sleeve, extending between the first axial end and the second axial end, so that the interior molding space is protected.
In the axial ends or end parts for such connected/closed state, a first annular encircling part and a second annular encircling part are provided, which are each defining a minimum sleeve inner diameter, preferably equal to the tube outer diameter. A given inner diameter can be obtained at the axial ends, which is suitable for contact with each corresponding fluid conveying flexible tube passing through the sleeve.

Each sleeve can have expanding and contracting means, which are configured to have each sleeve part expanded in a by-default configuration, but allowing a reduction in an inner diameter of the sleeve, when the sleeve is submitted to a squeezing and/or to a compression between two plates or similar opposite parts of the holder assembly that receives the two sleeves between these two plates or similar opposite parts.
The cutting member involved for the cut can pass, as in known devices, through a passage separating two pressing subparts provided in the two plates/parts that perform the squeezing/clamping.

More generally, the tube portion extending between the two axial end parts can be maintained between the first annular encircling part and a second annular encircling part, these encircling parts typically protruding radially inward, from the inner face, more than the pins or protrusions. The sleeve encapsulation can involve/include:
- a first encapsulation step, performed with a hollow, at the interior molding space, until a new junction is obtained by the cut and connection method, which is a junction between two cut end faces of tube segments that were initially distributed in the two different tubes;
- then a second encapsulation step, with an injection of the curable material to cover the junction, possibly using an injection port.

Alternatively, the curable material is kept protected, for instance by a peelable membrane or similar protecting member, along the inner face so that the hollow or molding space is already filled with the curable material.

An injection port for filling a flowable material (forming the curable material) can be provided in the sleeve, with a radial channel included in the injection port. More generally, access to the cavity/interior molding space or inner recess, through material thickness, can be provided. The injection port can include an inlet opening outwardly and an outlet opening inside the cavity/molding interior space.

Typically, the kit for encapsulated connection can be provided with the curable material that is preferably flowable and configured to cover each inner face and be cured after the cut and the rearrangement. In options:
- the curable material is silicon, possibly with the curable material maintained or configured as a layer covering an inner face of each sleeve.
- the curable material can be any suitable material flowable and able to be cured under a treatment chosen amongst a heating, an irradiation (UV irradiation for instance) or a combination of these operations.
- the curable material is flowable and can fill inner grooves or similar hollows of thickness reduced areas, provided in each sleeve/each sleeve part.
- each sleeve is provided with thickness reduced areas, interposed between distinct series of pins, the thickness reduced areas extending parallel, each from one of the two axial ends to the other one of the two axial ends.

In some embodiments of the kit, one or more of the following arrangements/features can be provided:
- the first sleeve and the second sleeve are two identical pieces,
- each sleeve has a longitudinal hinge.
- each sleeve is provided with interlocking members at the linking area.
- the two sleeve parts are two halves that can be cut at a midsection thereof by a same blade or cutting member involved for the cut of the two flexible tubes.
- the kit can comprise or be associated with two tubes/the two tubes.
- the tubes preferably have a same inner diameter and a same outer diameter.
- the two sealing sleeves are a first sleeve and a second sleeve that are recessed.
- the two sleeves have same inner diameter, preferably defined at two opposite encircling ends.
- the encircling ends may be slotted or provided with teeth.
- a region part of a tube amongst the first tube and the second tube can be maintained between the first annular encircling part and a second annular encircling part.
- a clip connection is established to have each sleeve firmly secured to a tube with the sleeve continuously surrounding the tube (without interruption).
- the interlocking members comprise two tabs or protruding latches arranged on a first connection surface, while recesses are provided on a second connection surface.
- the first and second connection surfaces are in abutment at a junction.
- the interlocking members are arranged outside (entirely outside) the interior molding space.
Preferably, the junction is provided opposite a longitudinal hinge of the sleeve. In some options, the interlocking members are arranged on both a first and a second connection surface of each of the sleeve parts (possibly parts constructed as halves forming a half circumference for each sleeve).

While the sleeves can be identical (in shape, in size and possibly regarding the deformable material used), they can also (in a variant) have a difference in length or in some details, but they may have the same inner radius and preferably the same arrangement of pins/protrusions and same axial end parts formed as constrictions. The interlocking members may be provided without any reliefs protruding radially outside (on outer surface of the sleeve), once the interlocked state is obtained.

The kit can be used for a given range of outer diameters of the tubes, which are (of course) tubes having an outer diameter lower than an outer diameter of the sleeves when the sleeves have a circular outer cross section (in undeformed configuration). The tubes are typically mono material, for instance entirely made of silicone.
Besides, each sleeve (the first sleeve and the second sleeve) can be provided with an injection port for a filling of the curable material through a sleeve external wall. Possibly, the injection port can cross a hinge area where two sleeve parts are connected.

Optionally, the kit comprises an injection tool (for instance a syringe provided with a piston or an injection actuation member) filled with the curable material. The injection tool can be provided with an injection tip configured to pass through sleeve thickness or via an injection port. In some embodiments, the sleeves each have a cylindrical outer wall without any side opening and/or without any injection port.

The sleeves of the kit can be thermoresistant, for instance made of thermoresistant silicone and/or resistant (not immediately degraded and/keeping its structure) at a temperature of 150°C. The encapsulated connection kit can already have a curable material (for instance silicone or curable material including at least 50% silicone) filling all or part of the interior molding space.
Typically, at least one amongst the first sleeve and the second sleeve is provided with a layer of the curable material that is kept along the inner face and preferably covered by at least one peelable membrane (or similar containment part or sheet). When such curable material is already present, two initially distinct layers can be formed, in the respective two parts of the sleeve that can be interlocked. These two layers allow covering the inner face in the assembled state of the sleeve.
The first and second sleeves are protecting sleeves to allow protection of an encapsulating and sealing/bonding layer: proper sealing of each tube connection at periphery of such connection is obtained, at least when the sleeves are recombined (in a recombined configuration) with the curable material being cured and forming the continuous bonding layer filling a collection zone around the new tube contacts. The collection zone combines two subareas of each interior molding space and remains delimited by sleeve (rearranged sleeve) having the same properties and structure as in the sleeve plastic pieces forming the interlocked sleeve parts.

The encapsulated connection kit according to any one of the preceding claims, wherein in each of the first sleeve and the second sleeve, the sleeve parts (typically two sleeve parts) are deformable, preferably without modifying general orientation of the pins or protrusions. The two sleeve parts can comprise, each:
- inner grooves that extend parallel to the central axis.
- hollows or inner grooves (possibly the parallel inner grooves) that are spreading the corresponding sleeve part in an undeformed configuration (by-default configuration).
- a retracted configuration due to areas of reduced thickness, for instance thanks to the inner grooves that can be narrowed and/or have a small hinge effect.
- an accordion structure, for instance with the inner grooves (parallel/longitudinal grooves) spaced by intermediary portions to limit the folding, the pins or protrusions being provided in the intermediary portions.
More generally, each sleeve can be deformable upon a pushing action exerted radially inward.

In some optional embodiments, each of the first sleeve and the second sleeve, made or consisting of a plastic material more rigid than silicon, is squeezable in a squeezed state such that each of the inner grooves is closed or reduced (in width) in the squeezed state, while the pins or protrusions that include said plastic material are rigid and configured to keep their size and shape in the squeezed state.
Possibly, the pins or protrusions are forming a row (of at least four or five protrusions/pins) that extends parallel to the inner grooves.
In some options, the plurality of pins or protrusions comprise one or several rows of at least nine pins or protrusions distributed circumferentially around the central axis (each row being perpendicular to the inner grooves when such grooves from the spreading and contracting means).
in variants, the protrusions are hooks, hollow pins or embossments, or are formed with a loop construction or handle-shape
In some options, there are more reliefs near a cutting site than reliefs away from the cutting site.

In some preferred embodiments, the two opposite axial end parts are provided with interspaces (formed as V-shaped gaps for instance), possibly interspaces between teeth with a variable spacing size due to deformation of the sleeve. Such interspaces are distributed in each sleeve part for the first sleeve and the second sleeve. Typically, in each of the first sleeve and the second sleeve in undeformed configuration, the two sleeve parts can each comprise:
- first interspaces regularly distributed at different angular sectors and forming part of a first subgroup of the interspaces (formed between the teeth), which are provided in one of the two opposite axial end parts.
- second interspaces regularly distributed at different angular sectors and forming part of a second subgroup of the interspaces, which are provided in the other one of the two opposite axial end parts.
Besides, each sleeve can have respective grooves of the inner grooves, which are each in alignment with:
- an interspace of the first subgroup, and
- an interspace of the second subgroup.

Possibly, all the inner grooves are in alignment with two corresponding interspaces of the two subgroups. When the two opposite axial end parts, formed as constrictions, each comprise teeth that protrude radially inward, the respective interspaces can decrease with increased pressure applied on the sleeve to close the lumen in the tube surrounded by the sleeve and squeezed by the pins or protrusions. More generally, any suitable interspaces (provided in undeformed configuration of the sleeve) between the teeth or similar end protrusions can be closed together with the inner grooves or similar spreading elements of the sleeve parts, which can be retracted due to the external pushing to be exerted before a cut. In the kit, each of the sleeves can have the respective interspaces with to a V cut or V-shaped slot (with a regular distribution in the axial end parts of the sleeve, which are provided to contact the tube and allow closing the interior molding space).

According to another aspect, embodiments provide a cutting and connecting device for cutting two tubes by simultaneously cutting a first flexible tube at a tube section thereof and a second flexible tube at a tube region thereof, and then establishing a pair of connections by fixing a first tube end of the first flexible tube, obtained by the cutting, to a tube cut end of the second flexible tube and also fixing a second tube end of the first flexible tube, obtained by the cutting, to another tube cut end of the second flexible tube, so that a rearranged tubing fluid connection for a biopharmaceutical fluid is formed,
wherein the device, which comprises the kit of two sealing sleeves as above presented to form a first sleeve surrounding the first tube at the tube section and a second sleeve surrounding the second tube at the tube region, further comprises:
- a tube holder assembly having a first tube and sleeve holder and a second tube and sleeve holder that are adjacent along a separating plane and are forming together a pair of receiving housings to have the first sleeve and the second sleeve maintained parallel with an interspace and each perpendicular to the separating plane;
- clamping parts included in or coupled to the tube holder assembly and allowing each sleeve to be squeezed between one of the tube and sleeve holders and a clamp member of the clamping parts;
- a cutting member configured to pass between the first tube holder and the second tube holder, along the separating plane, the cutting member being movable to cut the first sleeve and the second sleeve when passing in/through the interspace (Sp);
- a driving unit allowing to reduce a given size of each receiving housing, along a given clamping direction by a clamping action using the clamping parts, preferably simultaneously, so that two fluid free areas are obtained where the clamping parts are in a clamping configuration against the first sleeve to squeeze the tube section and against the second sleeve to squeeze the tube region; and
- a movable support that supports one amongst the first tube and sleeve holder and the second tube and sleeve holder, on a given same side relative to the cutting member, the movable support being configured to rotate at an angle of 180°, on said given same side, to displace the first tube & sleeve holder or the second tube à sleeve holder correspondingly;

wherein at least one amongst the first tube and sleeve holder and the second tube and sleeve holder is displaced by the driving unit to allow the first tube and sleeve holder and the second tube and sleeve holder to be axially closer after a removal of the cutting member and obtain said pair of connections,
and wherein the resiliently deformable plastic material of each of the first sleeve and the second sleeve allows, upon releasing of the clamping configuration, to have:
   - two rearranged sleeves of same cross-section as the first sleeve and the second sleeve with a circular section around the corresponding tube; and
   - the pins or protrusions coated or being configured to be coated by the curable material.

With such device, the operator can obtain a controlled junction (with a suitable bonding layer formed beneath the corresponding wrapping part), possibly keeping asepticity without complicated steps. There is no need to clean and reuse a mold since the molding cavities are embedded in the kit, with each molding sub-cavity (obtained after the cut) compatible with a squeezing-release cycle due to the deformable (preferably resiliently deformable) material of the sleeves. The operator has only to form the sleeves, for instance with a clipping to snap the sleeve parts around each corresponding tube, and then place the assembled sleeves in the tube and sleeve holders. The sleeves are advantageously transmitting the clamping action, via the pins or protrusions, preferably circumferentially along the tube area provided with the cutting site.

For biopharmaceutical use, the device facilitates the operation to handle the two collapsible tubes, using sleeves constructed as pinching sleeves. Rearrangement and cut of the sleeves do not involve any additional step as compared to situation where the tubes are directly pinched by tube holders and cut transversely. This is of interest to have each bonding layer encapsulated (beneath the wrapping parts of the sleeves) around the reconnected tube section/region.

The tube section and tube region can thus be cut via a transverse cut (with the cutting member being guided in the device, optionally inside a casing of the device) that is also cutting the two sleeves at the respective cutting sites. The gap between the holders may form a passageway allowing insertion of the cutting member to cut the two tubes and the two deformable sleeves, due to a relative movement between the cutting member and the housing set delimited by the holders. It is understood the tube section/tube region can be deformed radially when maintained in the housing, in the closed conformation (locally emptied tube), while being kept stationary at least along an axial direction parallel to each central axis of the sleeves.

Practically, each tube and sleeve holder can optionally be only in contact with the four sleeves parts, on one side relative to the separating plane. The first and second tube and sleeve holders can each have a H cross-section to define two housing parts. Before the cut, a set of two housing parts, possibly forming a left set, is used to guide and maintain the first sleeve assembled around the first tube, while another set of two housing parts, so-called right set, is used to guide and maintain the second sleeve assembled around the second tube.
Of course, after rotation to rearrange the tube segments, the housing parts are rearranged differently: with one the right housing parts in alignment with the housing part rotated from the left side to the right side, while the housing part remaining on the left is now in alignment with the housing part rotated from the right side to the left side.

In embodiments of the device, the first tube and sleeve holder (first holder) comprises:
- first axial abutment means to prevent the first sleeve axially leaving the corresponding receiving housing along a first direction so that a first axial retaining is exerted by the first axial abutment means on the first sleeve with the first flexible tube that extends beyond the first axial abutment means.
The first holder can also comprise, optionally, second axial abutment means to prevent the first sleeve axially leaving said corresponding receiving housing along a second direction opposite from the first direction so that a second axial retaining is exerted by the second axial abutment means on the first sleeve with the first flexible tube that extends beyond said second axial abutment means and beyond two opposite axial sides of the tube holder assembly.

Preferably, the second holder is configured rotatable relative to the first holder.
Typically, the second tube and sleeve holder (second holder) also comprises similar abutments means (exactly as the first holder), with first and second axial abutment means to hold (axially hold) the second sleeve in the corresponding receiving housing together with the second flexible tube that extends beyond the axial abutment means and beyond two opposite axial sides of the tube holder assembly.
Besides, the first and second holders can either pinch the sleeves, or form groove-like receiving housings with the sleeves extending partly outside the grooves to allow a pinching by a clamp member squeezing the sleeve from the open side of the groove/receiving housing.

The driving unit can be configured to drive an axial movement to modify position of the first holder and the second holder in the tube holder assembly, these holders being spaced to allow the cutting member passing in-between, with the driving of the driving unit also allowing a junction at a midsection of each of the two rearranged sleeves, thanks to the first axial abutment means and the second axial abutments means that are engaged with respective sleeve ends away from a cutting plane (which may be the separating plane) along which the cutting member is displaced.
The first axial abutment means can comprise two axial inner shoulders of a first pair to respectively hold the first sleeve in the corresponding receiving housing together with the first flexible tube that extends beyond the two axial inner shoulders.
Two other axial inner shoulders of a second pair are provided to respectively hold the second sleeve in the corresponding receiving housing together with the second flexible tube that extends beyond said two other axial inner shoulders.
Typically, the axial inner shoulders of the first pair and of the second pair can be axially spaced along a tube direction that is perpendicular to said given clamping direction and perpendicular to said separating plane.

The movable support may rotate around a longitudinal axis/direction in response to a command of the driving unit. In some embodiments, a control unit may be provided to command a predetermined sequence of the steps, from the squeezing of the tubes each with their corresponding sleeve to the relative rotation between the first tube holder and the second tube holder and the subsequent axial junction (with the pair of tube connections) with removal of the cutting member.

Optionally, the cutting and connecting device further comprises a welding unit, possibly provided with temperature increase means, configured to perform junction of the two tubes, which are preferably silicone tubes, by curing the curable material, preferably by a heating operation or UV irradiation exerted/transmitted through each of the sleeves, along a circumference around:
- the tube section; and
- the tube region.
The temperature increase means may be of the type involving heat conduction or using an IR source or similar irradiation source to cure the material involved for the welding performed by the material layer present in the cavities/interior molding spaces.

the device may allow cutting of the tube section and cutting of the tube region to divide the fluid free area of the tube section into two subareas and the fluid free area of the tube region into two other subareas, while the tube section and the tube region are maintained in the pinched state in the tube holder assembly. A first tube cut end and a second tube cut end are separated by the cutting member at the tube section, in the clamping configuration. The cutting member has an extension configured to also separate two other tube cut ends at the tube region.
Besides, compression of the tube and sleeve assembly can be obtained by placing this assembly between two jaws of the device, whereby a mechanical sealing of the tube region is obtained by the appliance having the two jaws maintaining the sleeve in a squeezed state.

According to a further aspect, it is provided a method of joining a first fluid conveying plastic tube to a second fluid conveying plastic tube equal in inner diameter and/or outer diameter to the first tube, while also joining a third fluid conveying plastic tube to a fourth fluid conveying plastic tube equal in inner diameter and/or outer diameter to the third tube, the method comprising:
(a) providing a first continuous tube and a second continuous tube, one of which initially includes the first plastic tube and the third plastic tube formed as two complementary segments of the first continuous tube, the second continuous tube including the second plastic tube and the fourth plastic tube formed as two complementary segments of the second continuous tube;
(b) wrapping locally at a tube section the first continuous tube, by a first sleeve, and also wrapping locally at a tube region the second continuous tube, by a second sleeve, the first sleeve and the second sleeve being the two sleeves of the kit as above described;
(c) squeezing, by a tube holder assembly provided with clamping means, the tube section and the tube region by clamping, preferably simultaneously, the first sleeve and the second sleeve with the pins or protrusions (14) maintaining a spacing in each of the interior molding space (MZ) between the inner face (Fm) and the corresponding tube (T, T');
(d) performing the joining after a cut through the two sleeves, with each of the tube section (Ts) and the tube region (Tr) simultaneously cut while the two pins or protrusions (14) of the sleeves maintain a squeezed state of the tube section (Ts) and the tube region (Tr).

The joining may be performed with an apparatus that combines the cutting and the junction, for instance with a single cutting member (blade or the like). With a blade contact maintained just before establishing the junctions, the rearranged tubing fluid connection is formed with an annular junction providing an inner surface of substantially continuous inner diameter. The release of the squeezed state may be deferred, occurring after the junction by the curing material. Alternatively, the release may be performed at least before the curing operation.

Advantageously, the cut (i.e. tube disconnection) and reconnection process can be advantageously performed at any suitable location (no need for pre-installed devices), and there is no open connection created, while operations can be performed in a way that is user-friendly, without needing to operate within a controlled environment for the cut and rearrangement to establish the pair of connections.

With such a method, the local emptying of the tubes may be performed efficiently, and a single and/or fast operation may be involved for the squeezing and the cut, since the interspace between the tube holders provides suitable access for a cutting member. Typically, the tube is flexible, forming a collapsible tube to allow sealing a lumen of the tube. The method may apply to a tube having a single lumen or to a double lumen tube.

According to embodiment of the method, the squeezing of the two sleeves of the kit is performed in a same squeezing step, preferably in a same device or apparatus, and following steps or features can be provided:
- the tube holder assembly and a cutting member are used to transversely cut, along a cutting plane, the first and second continuous tubes at a cutting step, while the squeezed state of the tube section and the tube region is maintained by the tube holder assembly.
- the squeezed state is maintained via a pressing contact transmitted via the pins or protrusions.
- the cutting along the cutting plane is performed to: separate the third fluid conveying plastic tube from the first fluid conveying plastic tube; and separate the fourth fluid conveying plastic tube from the second fluid conveying plastic tube.
- the joining is performed, inside a pair of receiving housings of the tube holder assembly, in an aseptic manner (of course the receiving housings receive the sleeves and the holders of the tube holder assembly may be only in contact with the sleeves around the respective cutting sites).

The joining can be performed:
- after a relative rotation to have a first plastic tube cut end in alignment with and facing a second plastic tube cut end, along a direction perpendicular to the cutting plane to allow a first annular contact junction, and also a third plastic tube cut end in alignment with and facing a fourth plastic tube cut end, along a direction perpendicular to the cutting plane to allow a second annular contact junction; and
- after displacing the cutting member away from a cutting site, by immediately performing the first annular contact junction and the second annular contact junction, with the curable material provided to be cured in each interior molding space around respectively the first annular contact junction and the second annular contact junction. The rotation can be a rotation with an angle of 180°. After the removal/displacement of the cutting member, the joining can be performed, while maintaining a squeezed state without any axial gap at the cutting site.

In some options, one or more of the following dispositions are provided:
- the tube holder assembly is provided with two holders, forming a first holder and a second holder that are arranged in alignment along a longitudinal direction that is perpendicular to the cutting plane.
- two sleeve receiving cavities are each distributed in the first holder and in the second holder, the sleeve receiving cavities being a left cavity and a right cavity.
- the first sleeve is housed, at a first side (left side for instance) of the tube holder assembly, in a first holder and in a second holder, while the second sleeve is housed, at a second side (right side for instance) of the tube holder assembly in the first holder and in the second holder.
- each of these holders can have a pair of cavities or grooves spaced by a gap that can form the interspace between the two receiving housings of the holder assembly (the cavities or grooves forming each a housing part on a given side relative to the separating plane).
- the tube holder assembly has the first holder and the second holder constructed adjacent along a cross/separating plane so that the first sleeve and the second sleeve are maintained parallel, each perpendicular to said plane with an interspace.
- the cutting along the cutting plane is performed along or parallel to the separating plane with a cutting blade that passes through a slot that separates the first tube holder and the second tube holder.
- the tube holder assembly, provided with the holders, is carrying a cutting member that can optionally pivots around an axis parallel to the longitudinal axis (parallel to the sleeves).
- the tube holder assembly is used to allow the sleeves and the tubes to be transversely cut, along a same cutting plane to provide four cut end faces in the first and second continuous tubes.
- the cutting member is interposed between the two holders that are in alignment so that: two cut end faces are aligned simultaneously with alignment of the two other cut end faces, while keeping the four cut faces closed by the cutting member.

In preferred embodiments, the slot can be formed temporarily, by displacing one of the holders axially and creating an axial space. The slot can disappear as soon as the cutting member is moved (typically back to a retracted position, away from the holders that guide and maintain the rearranged sleeves). Besides, the method typically comprises:
- withdrawing the cutting member from the cut end faces (thus from the cutting site), and immediately bringing each of two pairs of the cut end faces into mutual tight contact, whereby that can be welded indirectly via the curable material (possibly with a curing treatment performed after the mutual tight contact).
The mutual tight contact can be maintained, using a pushing force exerted axially on at least one of the holders, with abutments parts driving the sleeve end axial parts to move/push them axially.

In embodiments of the method, the kit can be used according to one or more of the following options:
- for a cut and rearrangement operation performed by the cutting and connecting device as above described.
- for keeping the tubes each in wrapped state with the sleeves kept assembled around them, with knowledge that the first continuous tube and the second continuous tube are, after being coupled to the two sleeves and being cut together with the sleeves: encapsulated by the curable material that is filled along each tube to circumferentially cover the cut tube ends.
- for allowing the curable material to form a pair of continuous layers, of tubular shape, beneath covering parts of the sleeves.
- for establishing silicone tube connections, with the curable material possibly including silicone.

The tubes, which are thermoplastic tubes or silicone tubes, are firstly coupled to the two sleeves and then cut together with the sleeves inside the respective receiving housings of the device. The number of tubes can be two or more. Permutation is allowed with any number of tubes.
Optionally, the curable material is provided in the form of an inner layer along each inner face of the respective sleeves, before the cut and rearrangement operation. Such inner layer can be protected by one or more membranes that cover(s) the interior molding space, possibly a membrane that is pullable to be removed after the assembling of the sleeve around the tube and after a squeezing but before any cutting of the tube. The cut and rearrangement is performed so that rearranged tubes are still wrapped (with a different combination of the sleeve parts, due to the rearrangement).

After the cut, the sleeve parts which are assembled to form a sleeve are each split into two subparts. Of course, each of the subparts of the sleeve parts/halves can have same inner diameter, possibly due to the fact that the sleeve parts have side wall with a symmetry cross plane at a middle thereof.
An assembling step can be obtained by use of two sleeve parts/halves provided with foolproof means to allow only one assembled state. Possibly, a rotation is involved I the assembling step, using a hinge that interconnects the two sleeve parts. The sleeve can consist of two sleeve parts/halves and can be provided, in assembled state, with a first annular encircling part and a second annular encircling part that are each defining a minimum inner diameter, preferably equal to the tube outer diameter, whereby a region part of the encircled tube can be maintained between the first annular encircling part and a second annular encircling part.

### BRIEF DESCRIPTION OF THE DRAWINGS

The figures of the drawings are now briefly described.
Fig. 1 schematically illustrates steps involving a kit with two sleeves according to an embodiment, until cutting a pair of tubes that are each provided with a deformable sleeve assembled and wrapped so that a tube region including a cutting site and forming two adjacent areas on both sides of the cut can kept pinched after the cut, upon a squeezing applied on the sleeves.
Fig. 2 is a perspective view of one embodiment of a reconnection allowed with two tube portions remaining wrapped by two wrapping parts recombined from a wrapping part of a first sleeve and another wrapping part of a second sleeve, which can be the sleeves of the kit used in the steps illustrated in Fig. 1.
Fig. 3 is a top view of one embodiment of a biowelding apparatus that may be used in accordance with the present disclosure, to perform the squeezing step, the transverse cutting step and the rearrangement step..
Fig. 4 shows, in a longitudinal cut view, an exemplary embodiment of a pinched configuration obtained for a tube and an associated sleeve that are maintained in the pinched state by two holders of a tube holder assembly, just before a cut with a spacing maintained around the pinched region of the tube to have an interior molding cavity for a curable material.
Fig. 5A illustrates a first embodiment of a separable sleeve part that constitutes a half of a sleeve, adapted to be coupled with an identical or similar half to delimit an interior molding cavity, axially between two sleeve end parts.
Fig. 5B illustrates a simplified view (tube not shown, tube holder not shown) of the sleeve formed as an assembly of two sleeve parts, in a pinched configuration with tightened end flange.
Fig. 5C illustrates, during the pinching or after releasing the pinching and with the wrapped tube remaining transparent, the interior molding space being filled with a curable material, to create a covering layer that ensures a continuous sealing contact around the new tube connection.
Fig. 5D illustrates an option for allowing a direct clamping by the front and rear tube holders of a tube holder assembly, configured to contact the tubes and axially block the sleeve end parts, before and after the rearrangement that involves a 180° rotation of one of the holders.
Fig. 6 shows a flowchart with steps of a method using the kit of two sleeves according to an embodiment, from the cut to a sealing material curing step ensuring that each of the two new connections concealed beneath each rearranged sleeve remains an aseptic connection.
Fig. 7 is a detail view of an exemplary arrangement with teeth formed at each sleeve end part, here for a by-default configuration (not pinched) of the sleeve.
Figs 8A, 8B, 8C, 8D show respective examples, with different geometry, for protrusions or pins that are formed on the inner face of each sleeve part.
Fig. 9 is a detail view of a sleeve that carries a layer of the curable material, typically with at least one optional protection membrane entirely covering the pins/protrusions and the curable material, whereby the curable material can be present along the sleeve inner face before the pinching and before the cut.
Fig. 10 is a perspective view of sleeve in open configuration, according to a second embodiment with two sleeve parts, preferably inseparable, which are connected by a hinge structure.
Fig. 11 is a perspective view of one embodiment of a biopharmaceutical circuit, here with a cell culture apparatus, where the device of the present disclosure may be used.

### MORE DETAILED DESCRIPTION

A detailed description of several embodiments of the invention is provided below, accompanied with examples and with reference to the drawings.

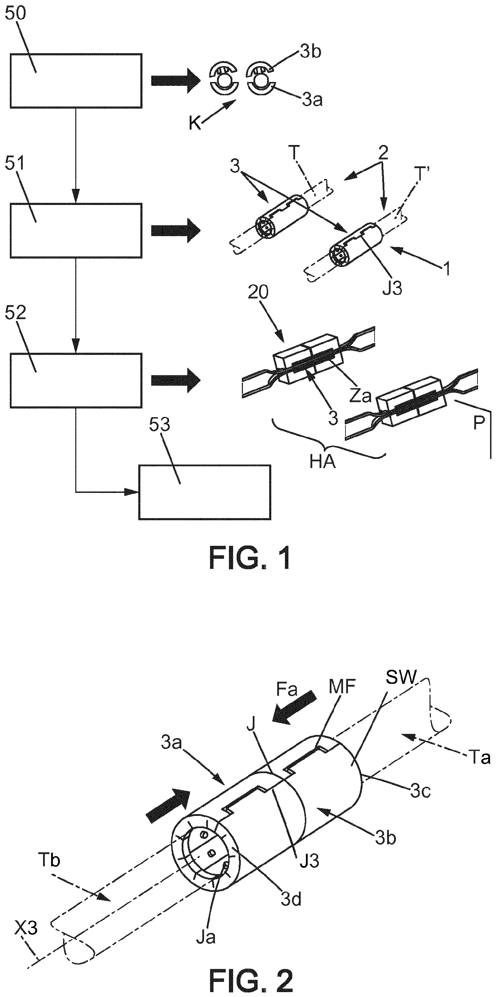

In the various figures, the same references are used to designate identical or similar elements. Some size or thickness may be exaggerated for the purpose of better illustration. For instance, spacing between the tube holders is exaggerated in Fig. 1 for the purpose of facilitating illustration of some functions and elements.

The embodiments described provide examples and should not be interpreted as limiting the scope of the invention. Features from one embodiment or aspect may be combined with features from any other embodiment or aspect in any appropriate combination. For example, any individual or collective features of method aspects or embodiments may be applied to apparatus, product or component aspects or embodiments and vice versa.

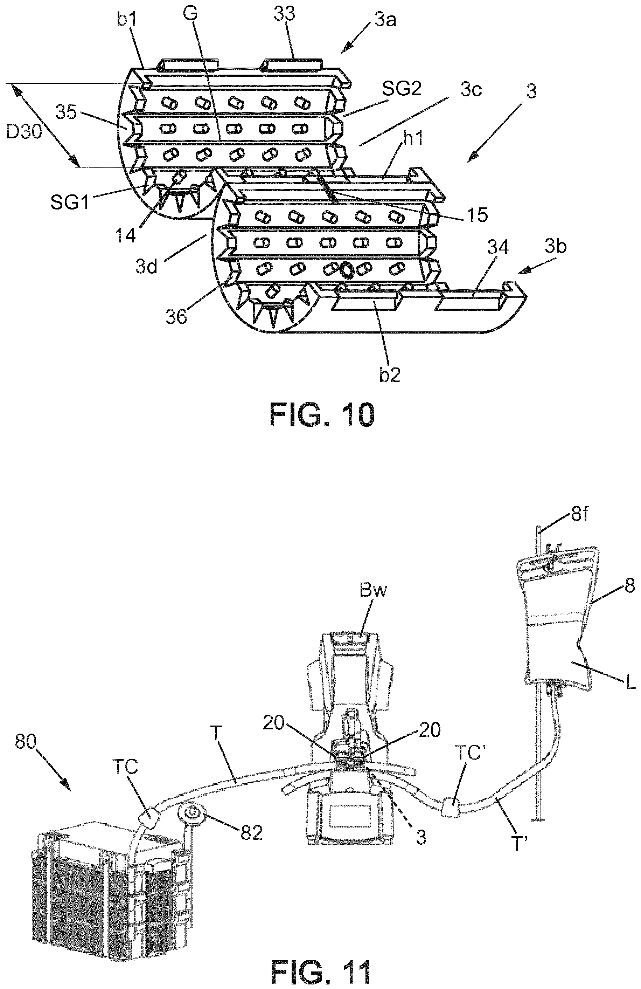

Referring to Figs 1-2 and 10, a kit K is provided with sleeves that each comprise sleeve parts 3a, 3b, the sleeves being used as a pair to be coupled to a pair of flexible tubes T, T'. The flexible tubes T, T' may have a constant nominal diameter, which can be similar or identical to an inner diameter of the sleeves once they are assembled, in an undeformed state of the sleeves. The sleeve parts 3a, 3b can be complementary plastic pieces or halves, typically only made of plastic/without any metal, and constructed to provide an open configuration. The sleeve parts 3a, 3b are either separate (as illustrated in Fig. 1) or may have an open state with at least one junction side keeping these sleeve parts 3a, 3b connected (possibly permanently connected) as illustrated in example of Fig. 10 with a hinge h1 forming such longitudinal junction in a one-piece construction.

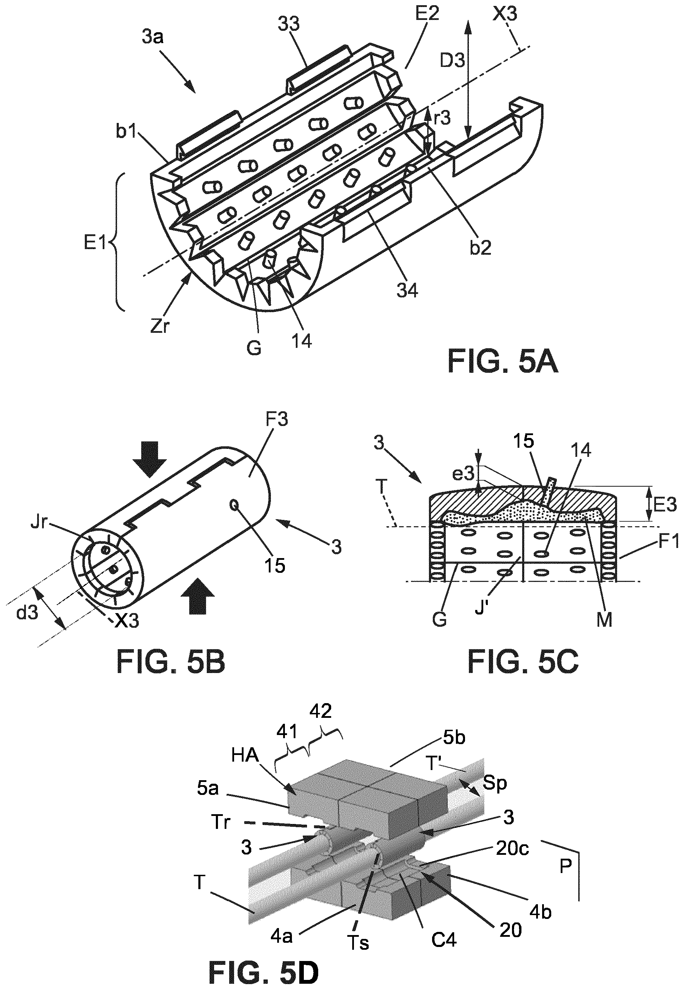

Each sleeve part 3a, 3b can be longer than wide. Thickness e3 of the sleeve parts may be reduced at a recessed part thereof, as illustrated in Fig. 5C for instance with a thickness E3 that is greater at the ends E1, E2 and/or increased. The increased thickness ends E1, E2 can form the end parts or end flanges in the sleeves 3, which may be:
- in radial contact with the tubs T, T',
- while also being in axial contact with abutments parts provided in the clamping means or holders involved for a tube cutting.

Lower thickness e3 of each sleeve part 3a, 3b may be optionally provided in middle portion thereof. Besides, at least one sleeve part 3a, 3b can be provided with an injection port 15 or similar channel that can be open for a filling operation with a curable material M, as described in more detail farther.

The kit can form an encapsulated connection kit provided with the two sleeves 3 (protecting sealing sleeves) for surrounding two flexible tubes T, T', typically in a biopharmaceutical circuit or assembly where such tubes are flexible and collapsible, with ability to be closed (with the tube lumen locally closed) upon a pinching action. As shown in Fig. 1, each sleeve 3 can have a length sufficient, for instance superior to 20 or 25 mm, so that in pinched configuration an elongated area ZA of the tube wrapped by the sleeve 3 is squeezed to ensure fluid is removed along this elongated area ZA.
The sleeve 3, 3' has pins or protrusions 14 that project radially inward from the inner face Fm, which may optionally be a concave/substantially cylindrical face due to the curvature of the sleeve parts 3 that are forming the tubular shape of the sleeve 3.

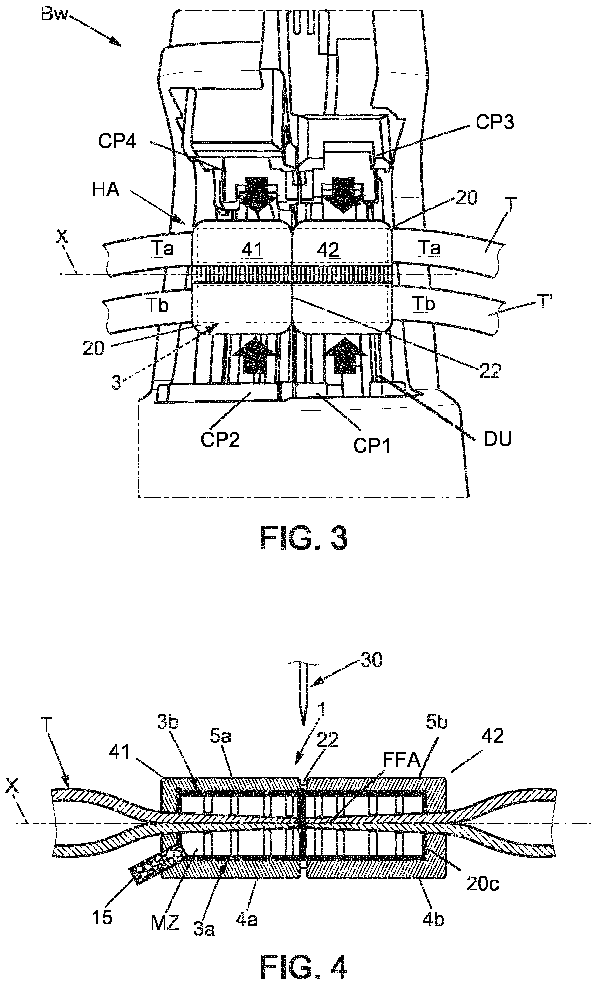

Each sleeve 3, 3' of the kit K not only wraps a tube T, T' and allows a squeezing action, but can be easily cut when cutting the tube T, T', preferably at a midsection of the sleeve 3. Referring to Figs 2, 4 and 9, the two sleeves 3, 3', which comprise each resiliently deformable plastic material in the sleeve parts 3a, 3b forming the external wall SW (Fig. 2) of tubular shape, can each delimit an interior molding space. In assembled state with the sleeve parts 3a, 3b interlocked, each tube T, T' passing through the central hollow formed by the assembled sleeve can extend rectilinear, due to the fact each assembled sleeve 3 extends tubular around a central axis X3. Locally, a longitudinal axis X of the tube T, T' can coincide with the central axis X3, as shown in Fig. 4 for instance.

Fig. 5B shows that the outer face F3 of each sleeve 3, once assembled, can have a rounded profile, facilitating a squeezing of the tube upon pushing/pressing action exerted from outside the sleeve 3. Possibly a guide or local relief can be provided for positioning of the assembled sleeve 3 in a tube holder assembly HA.
Also, each of the axial ends E1, E2 in the sleeve part 3a, 3b can have an undulated/wavy or toothed design Zr, typically with a regular distribution of areas with reduced thickness, for instance due to radial slots provided in the axial end (with each slot opening radially inward). The undulated or toothed design Zr can have an according effect, possibly in combination with inner grooves G that are formed, longitudinally, on interior surface of each of the sleeve parts 3a, 3b.

In assembled and undeformed configuration, each sleeve 3, 3' is provided with an inner face Fm that can be continuous (defining a substantially constant inner circumference). The inner face Fm may have a generally circular section so that each of the two sealing sleeves 3 has an inner radius r3 (Fig. 5A), as measured between the inner face Fm and the central axis X3 in the undeformed sleeve configuration.
This undeformed configuration, which is obtained when fixation means lock the assembled state, is compatible with pushing actions and squeezing operation, for instance with the cross-section of the sleeve 3, 3' being deformed (substantially flattened for instance) when using clamp members or constraining plate members that can reduce a size of the wrapped tube by reducing the corresponding size of the sleeve 3, 3'.

Each sleeve part 3a, 3b can have a constant length, measured between two axial ends E1, E2. Alternatively, or additionally, only the inner recess in the sleeve part 3a, 3b has a constant length formed as subspace of the interior molding space. This recess can be delimited between the axial ends E, E2.

The sleeve parts 3a, 3b can be each significantly deformed upon a pushing from the outer face thereof, for instance by having the inner grooves G (each parallel to the central axis X3) providing an accordion effect completed by the interspaces 36 between the teeth 35 at the axial ends E1, E2. As illustrated for instance in Fig. 5A, the two sleeve parts 3a, 3b, adapted to form a sleeve 3, can be compressed with the axial ends E1, E2 that can have a toothed design.
The teeth 35 may be spaced (with interspaces 36 as apparent in Fig. 10) in undeformed state with the nominal radius of curvature, while the interspaces 36 can disappear with reduction of the radius of curvature of the sleeve parts 3a, 3b as shown in Figs 2 and 5B with the tube(s) squeezed via the sleeve pinching. The interspaces 35 can be V-cuts so that the teeth 36 can get closer to form side junctions Jr (Fig. 5B), whereby the teeth can be part of a disc-shaped flange in pinched state. The reduction of the radius of curvature, in compressed state, is of interest to more tightly/tightly delimit each interior molding space MZ, axially. It is understood that any suitable design can be provided at these ends E1, E2, provided that each assembled sleeve 3, 3' can be deformable upon a pushing action exerted radially inward, so that the pins/protrusion 14 compress the tube T or T'.

Each sleeve part 3a, 3b may have a plastic material more rigid than silicon but squeezable, typically such that each of the inner grooves G is closed or reduced in the squeezed state. The pins or protrusions 14 cannot deform (or not significantly) upon such radial compression: they keep their size and shape in the squeezed state.

When the ends E1, E2 are formed as constrictions, each comprising teeth 36 distributed on half circumference, the respective interspaces 35 (between the teeth 36) are forming pairs that are in alignment with a corresponding inner groove G. In other words, for a given sleeve part, the interspaces 35 can have predetermined angular positions in the ends E1, E2, to have a first subgroup SG1 of interspaces (in the axial end E1) and a second subgroup SG2 of interspaces (in the axial end E2), with each having the same number of interspaces 36, which is equal to the number of inner grooves G that are formed in the sleeve part 3a or 3b.
The teeth 35 may be each longer than a depth of the inner grooves G, in order to form constrictions while the inner grooves G can be constructed by hollows or spaces in the thickness of the external wall SW.

As illustrated in Fig. 10, a nominal diameter D30, as measured for a halve/ sleeve part 3a, 3b (between the two opposite longitudinal sides thereof) or for the assembled sleeve 3 in undeformed configuration, can be defined at an inner edge defined by the free ends of the teeth. This nominal diameter D30 is lower than nominal diameter D3 (Fig. 5A; D3 is equal to twice the radius r3) of the inner face Fm.
In some options, the sleeve 3 is provided with at least eight inner grooves G, parallel and each having an elongated bottom line (extending along a direction D and acting as a hinge), and can be compressed sufficiently so that the inner nominal diameter 30 can be:
- greater than an outer diameter of the sleeve 3 as compressed in pinched state;
- and/or at least 20% greater than a reduced diameter d3 delimited by the same teeth in the pinched configuration such as illustrated in Fig. 5B for instance.

### Examples of interlocking of the sleeve parts to delimit the inner face

To allow fast assembling of the sleeve parts 3a, 3b, a clip connection can be established. Referring to Figs 2, 5A-5B and 10 using tabs 33 that are resiliently deformable or similar clips, provided on one the sleeve part 3a, which can engage with the other sleeve part 3b at a fixation region having a complementary shape or providing the complementary anchoring/detent members, with abutments for instance. When having one or two tabs 33 or protruding latches arranged on a first connection surface in the sleeve part 3a, recesses 34 can be provided on a second connection surface in the sleeve part 3b. More generally, interlocking members are used to keep the sleeve parts 3a, 3b firmly secured and assembled: this assembled state is thus locked. The interlocking members may be provided in the external wall SW, possibly without creating any inner relief on the inner face Fm.
Preferably, the assembling of the sleeve 3, 3' is performed only once the corresponding tube T, T' to be cut has been placed longitudinally along at least one of the sleeve part 3a or 3b, to then allow a wrapping step at the time of assembling the sleeve 3, 3'.

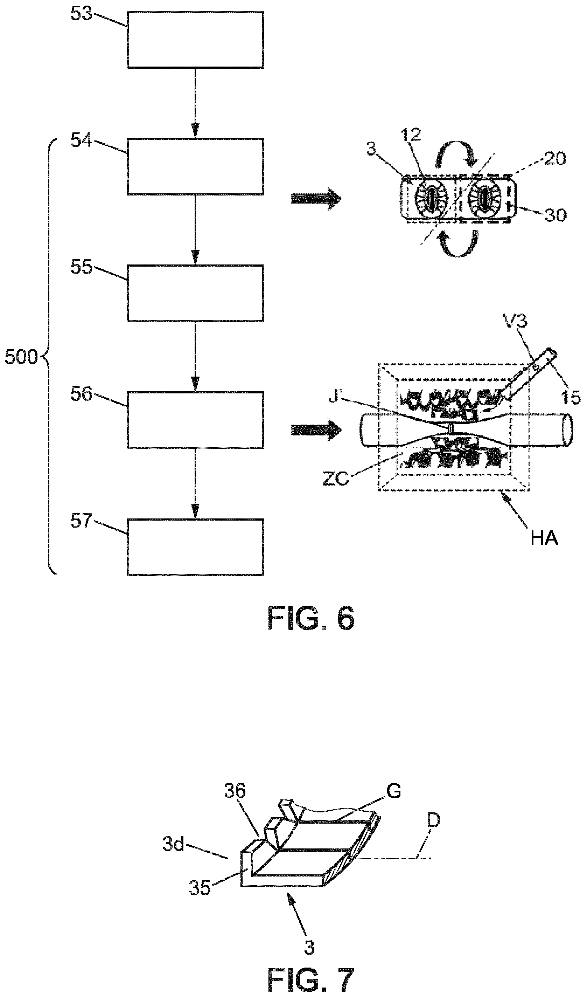

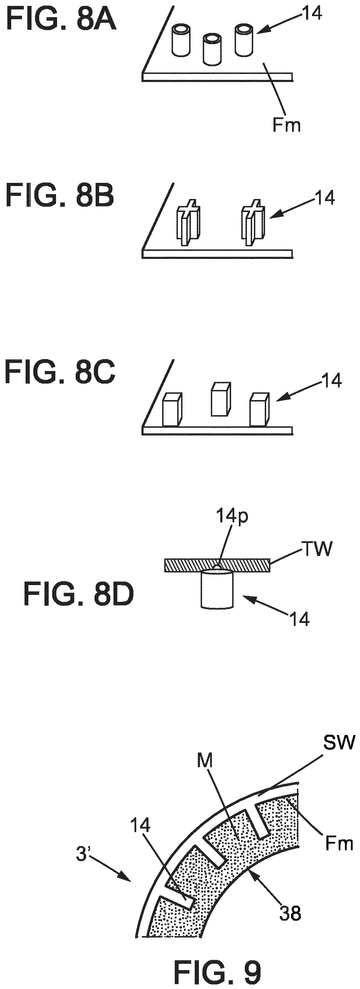

Each inner face Fm of a sleeve 3, 3' is provided with reliefs that can be distributed along length/axial size of a molding interior space. Practically in each of the sleeve parts 3a, 3b, pins or protrusions 14 can protrude radially inward, as well apparent in embodiments of Fig. 5A, 9 and 10 and for instance. Besides, Figs 8A, 8B and 8C show exemplary cross sections for such protrusions 14. Use of hollow shape, hollow cylinders is an option. A cross profile (Fig. 8B) or polygonal profile may be an option to define small pins that are robust. Possibly, the protrusions 14 carry a small tip or similar narrow end contact part to provide a hook effect and have the protrusion 14b partially inserted in the tube wall TW to have an anti-slipping effect.
Of course, each protrusion 14 is short. The pins or protrusions 14 can be shorter than the inner radius r3, preferably shorter than half the inner radius r3. The pins or protrusions 14 are configured to contact the tube T, T' and maintain a radial space between the tube T, T' and the inner face Fm, so that each interior molding space MZ can extend around the tube wall TW.
The pins/protrusion 14 can be coated with a curable material M that allows encapsulating and sealing each junction area (including the area ZA) where two of the tube cut end faces are axially joined, as illustrated in Fig. 6 for instance. In contrast, the interlocking members can extend outside the interior molding space MZ.
Referring to Figs 1 and 5A, non-limiting embodiments are shown, in which the first sleeve and the second sleeve of the kit K may be two identical pieces, possibly made separately and then assembled. Of course, a structure with halves can be provided without having the pieces strictly identical and/or initially separated. More generally, each sleeve 3, 3' can be provided with at least one linking area J3 that allows a full or partial disconnection, and reconnection, of two complementary wrapping parts/sleeve parts 3a, 3b. Each linking area J3 can correspond to a tangential junction in the external side wall SW that can be tubular with a junction contact, possibly a continuous contact, between two edges or connection surfaces b1, b2.
Each sleeve 3 can continuously surround the tube (without interruption). The first and second connection surfaces are in abutment at a junction, in assembled state. Preferably, a linking area J3 is provided opposite a pivoting connection or hinge of the sleeve. In some options, the interlocking members are arranged on both a first and a second connection surface of each of the sleeve parts (possibly parts constructed as halves forming a half circumference for each sleeve).

Each linking area J3 allows sufficient separation to have a tube T, T' insertable between the two sleeve parts 3a, 3b, at a tube intermediate portion that will be squeezed/closed, while having the two opposite tube outer portions (not wrapped tube portions) extending beyond the respective end parts 3c, 3d of each sleeve 3, 3'.
In option of Fig. 10, a hinge h1 forms part of fastening means to have the two sleeve parts 3a, 3b assembled to form the tubular-shaped sleeve. Two diametrically opposite linking areas can be formed: one at the hinge h1 and another one at junction between the two contacting surfaces b1, b2 that are distal from the hinge h1. In Fig. 10, it is understood that a 180° rotation allows a tube T or T' to be suitably positioned with radial contact with the protrusions 14 and wrapped by the sleeve 3 that reaches its tubular assembled configuration.

Now referring to Figs 1-4, it is shown embodiments where a tube T, T' is successively wrapped by an assembled sleeve 3, squeezed in a tube holder assembly that accommodates the sleeve 3, and cut by a cutting member 30 to be split in two tube parts Ta, respectively two tube parts Tb (see Fig. 3). The kit K is used to have the two tube T, T' cut and recombined, in a method that can produce:
- a recombined tube having a sealed connection between a tube part Ta from the tube T and a tube part Tb from the tube T', as illustrated in Fig. 2 with a rearranged sleeve still present but also having transverse connection J; and
- another recombined tube having the other tube part Tb connected to the other tube part Ta, also with a rearranged sleeve having the remaining sleeve cut portions of the kit K.
It is understood, such method allows each of the two tube parts Ta (with the corresponding outer tube portions) to be involved differently in a biopharmaceutical circuit.

### Exemplary use of the kit in a cut and rearrangement reconnection method

As illustrated in Fig. 1, it can be seen the kit K is used in method of:
- joining a first fluid conveying plastic tube (tube part Ta) to a second fluid conveying plastic tube (tube part b) equal in inner diameter and/or outer diameter to the first tube (Ta), while also joining a third fluid conveying plastic tube (other tube part Ta) to a fourth fluid conveying plastic tube (other tube part Tb) equal in inner diameter and/or outer diameter to the third tube (Ta),
the method comprising:
- providing the first (continuous) tube T and the (second) continuous tube T', one of which being the tube T that initially includes the first plastic tube Ta and the third plastic tube Ta formed as two complementary segments of the first tube T, the second tube T' including the second plastic tube Tb and the fourth plastic tube Tb formed as two complementary segments of the second tube T';
- placing (50) the sleeve parts 3a, 3b around each tube T, T', the sleeve parts being for instance four sleeve parts 3a, 3b so that two tubular sleeves 3 can be formed.
- performing a wrapping step 51 by wrapping locally at a tube section Ts the first tube T, by a first sleeve, and also wrapping locally at a tube region Tr the second tube T' (Fig. 5D), by a second sleeve, the wrapping step 51 including a locking operation by the respective interlocking members to obtain a pair 2 of pre-encapsulated tubes having each their respective protected tube section Ts/tube region Tr;
- performing a squeezing 52, by a tube holder assembly HA (typically provided with clamping means, as in Fig. 3 for instance), the tube section Ts and the tube region Tr by clamping, the first sleeve 3 and the second sleeve 3 with the pins or protrusions 14 maintaining a spacing in each of the interior molding spaces ZC between the inner face Fm and the corresponding tube T, T'.

The clamping at the squeezing operation 52 can be performed simultaneously for the tube section Ts and the tube region Tr, with the holder assembly HA to pinch each sleeve 3 and each tube T, T' at/from two opposite sides to close a lumen of the corresponding flexible tube T, T', under radial pushing effect of the plurality of pins or protrusions 14 as illustrated for instance in Fig. 4: It can be seen the sleeve parts 3a, 3b are compressed toward a median plane typically including the longitudinal axis X of the corresponding tube T.
The cutting step 53 can then be started, possibly using a cutting member 30 (with a blade for instance) that extends along separating plane P that separates two holders 41, 42 of the tube holder assembly HA.

Referring to Fig. 6, the sleeves 3 of the kit K can be cut when cutting the two tubes T, T', at the cutting step 53 with the cutting member 30 closing the four cut end faces of the two respective tubes T, T'. The cutting member 30 can be kept, substantially planar, in a position separating the two holders 41, 42. Then a reconnection and sealing operation 500 can start.

The reconnection and sealing operation 500 may comprise successive steps, including:
- an inversion 54 of two tube parts rotation to have two parallel tube parts Ta, Tb selectively inverted, preferably while the cutting member 30 remains in contact with the four cut end faces and typically with same in line disposition kept for the tube parts after the rotation;
- an axial contacting step 55, in which the cutting member 30 is removed along the separating plane P (which is also the cutting plane) to immediately allows a displacement for moving the holders 41, 42 closer and reach a face-to-face contact with obtention of new pairing of the four cut end faces, as schematically illustrated (Fig. 6);
- a molding/forming step 56 by forming an overlapping layer of the curable material M, which is a material injected or released to coat all or part of the pins or protrusions 14 and surround the new contact area to provide the connection J' (as illustrated in Figs 5C and 6 for instance);
- a curing step 57, for instance with heat curing or UV curing the material M, to have the bonding layer being a fastener in the connection J', with an annular shape of the bonding layer at least at the area of the connection J', between the two holders 41, 42.

In some embodiments, a rotation can be performed at the inversion step 54, using a movable support 12, to have new pairing of the four cut end faces as schematically illustrated (Fig. 6). The movable support 12, which can carry one of the holders 41, 42 for instance, can pivot with an angle of 180° while maintaining the two corresponding cut end faces that move in axial contact against the cutting member 30 that remains present along the separating plane P separating the two holders 41, 42.
In other words, the movable support 12 can support one amongst the first tube holder 41 and the second tube holder 42, on a given same side relative to the cutting member 30, with a rotation that can be controlled via a driving unit provided in a device Bw that can implement the steps of the above presented method to obtain the new pairing with the two connections J' beneath the rearranged sleeves.

In some variants, the device Bw can be a reconnection device that reconnects only a part of the cut ends, for instance only a pair of the tube cut ends, without any particular rotation (possibly with a sliding movement that is a translation).

### Embodiment(s) of a device for performing the new pairing

Referring to Figs 3 to 6, the kit K can be used to obtain the pair 2 of pre-encapsulated tubes having each their respective protected tube section Ts/tube region Tr. Then, a device Bw can advantageously make the operations easier for the operator, by performing at least some parts of the steps 54-57 of the reconnection and sealing operation 500 in automated manner.
During the curing of the material M, the molten plastic or silicon of this material M quickly solidifies leaving two sealed stub ends and one connected tube combining the tube parts Ta and Tb. The clamping parts CP1, CP2, CP3, CP4 or clamping halves 4a, 4b, 5a, 5b can then be opened and the wrapped tubes removed. The flow in each new tube can be reestablished, upon releasing the pinching: the elastic return of the sleeve 3 toward a circular cross section can help in having the lumen reopen, knowing that the tube is highly resilient to also return to the circular cross section.

Now referring to Fig. 3, after the wrapping 51 (preferably with assembled configuration locked, a device Bw may be used, which optionally operates with similar steps as compared to the process of the available apparatus Biowelder^{®} of Sartorius (fully automated device for connecting a set of two tubes of a thermoplastic tubing in a sterile welding operation / allowing sterile connection of liquid filled tubing).
Typically, the device Bw is a cutting and connecting device that is configured to cut the two tubes T, T' simultaneously, and then establishing a pair of connections J'. Unlike the Biowelder^{®} that heat the end cut faces, the device Bw can provide the connection by properly treating the curable material M and/or allowing the proper rearranged interior spaces to be available for forming a bonding layer via the curable material. The cutting member 30 may be rotatable, for instance with a retracted position at the rear of some clamping parts, similar to the position illustrated in Fig. 2 of WO 2010/118546.

The device Bw comprises a tube holder assembly HA provided with:
- cutting means including the cutting member 30 and, possibly, a central guide defining a cutting plane where the cutting member 30 is movable;
- a first tube and sleeve holder, forming the first holder 41, which may be designed to support two respective tube part Ta and Tb in parallel, adjacent to a cutting site that coincides with the cutting plane; and
- a second tube and sleeve holder, forming the second holder 42.
The two holders 41 and 42 are adjacent along the separating plane P (coinciding here with the cutting plane). Each holder 41, 42 can have a H profile (as in Fig. 3) to form each a single block. Alternatively, as in the case of Fig. 5 each holder 41, 42 can be split into two clamping halves 4a, 5a or 4b, 5b (Figs 4 and 5D).

When having the first and second holders (rigid holders), preferably made each of one rigid block with half housings formed on the two sides (two side grooves), the device Bw can have a large cavity or recess, as shown in Fig. 3, to provide the tube holders 41, 42 inside this cavity:
- in alignment along a first direction that is parallel to each axis of the tubes T, T' in the tube section Ts and tube region Tr (region that are wrapped each by a sleeve 3),
- arranged between clamping parts CP1, CP2, CP3, CP4 that allows the squeezing of the sleeves 3 (assembled sleeves) and, incidentally the squeezing of the tube section Ts and of the tube region Tr by the respective pins or protrusions 14 distributed circumferentially and longitudinally in each sleeve inner face Fm.
When having the clamping parts directly formed by the holders 41, 42, a cavity can be delimited between clamp members 5a, 5b and the clamp members 4a, 4b that also hold the sleeves 3 by having each a recess C4.

More generally, compression of each tube and sleeve assembly can be obtained by placing this assembly between two jaws of the device Bw that can form all or part of the corresponding tube holder 41 or 42, whereby a mechanical sealing of the tube region to be cut is obtained by the appliance/device having these jaws, with the sleeve 3 maintained in a squeezed state.

Whatever the specific construction of the tube holder assembly HA, a pair of housings 20 can be formed to receive the sleeves 3, 3' of the kit K, possibly with axial abutments parts 20c that prevent each sleeve leaving the corresponding housing. Such axial abutments 20c allow driving at least one of the holders 41 , 42, axially, when reaching the double contact at the axial contacting step 55.

The cutting means, here provided with the cutting member 30 or blade, is suitable to be actuated, possibly using a driving assembly functionally coupled to the tube holders 41, 42, once each wrapped tube has been mounted in the corresponding housings 20 and squeezed properly to have the two fluid free areas FFA, as in the option illustrated in Fig. 4 for instance.

The device Bw may allow the cutting of the tube section Ts and tube region Tr to divide:
- the fluid free area FFA of the tube section into two subareas,
- and the fluid free area of the tube region into two other subareas,
while the tube section Ts and the tube region Tr are maintained in the pinched state in the tube holder assembly. Accordingly, a first tube cut end (forming a cut end face) and a second tube cut end (forming another cut end face) are separated by the cutting member at the tube section Ts, in the clamping configuration. The cutting member 30 can have a geometry and size, in particular a height superior the nominal sleeve eight and a width sufficient, adapted so that the cutting member 30 also allows separating two other tube cut ends at the tube region Tr.

The device Bw can include a driving unit DU allowing to reduce a given size of each receiving housing 20, along a given clamping direction by a clamping action. In Fig. 3, the clamping parts CP1, CP2, CP3, CP4 can be used, possibly simultaneously, so that the two fluid free areas FFA are obtained where the clamping parts CP1, CP2, CP3, CP4 are in a clamping configuration against the sleeves 3 to squeeze the two tubes T, T' locally. The tube section Ts and the tube region Tr remain spaced, with a gap or interspace Sp formed by spacing means of the device Bw, for instance with a spacing structure included in each of the tube holders 41, 42 to delimit the housings 20.

The device Bw comprising rigid clamping members and/or rigid holders 41, 42, with the pair of receiving housings 20 delimited by surfaces suitable for the pinching, possibly with two substantially parallel planar portions in these surfaces. Before and after the squeezing step/operation 52, the first sleeve and the second sleeve received in the housings 20 are maintained parallel to the separating plane P with a constant interspace Sp, while these sleeves 3 remain each perpendicular to the separating plane P.
Referring to Fig. 4, it is understood the cutting member 30 is configured to pass between the first holder 41 and the second holder 42, along said plane P, with the cutting member being movable to cut the two sleeves simultaneously when passing in the interspace Sp. The shape and the dimensions of the cutting member 30 are selected in such a way that the cutting member 30 can-via the access or slot 22 (Fig. 4) at the interspace Sp -pass into the transverse passage for cutting the respective tubes T, T' into sections that are locally kept pinched.

The device Bw thus can pinch the two tube parts Ta and the two tube parts Tb after the cut and keep them closed, with the cutting member 30 providing a contact surface for allowing a sliding movement of cut end faces of some tube parts, typically one of the tube part Ta and one of the tube parts Tb, which are arranged on a same side relative to the cutting member 30. When these tube parts are rotated by the device Bw (biowelding device or similar cutting and reconnecting apparatus forming the device Bw) while their end cut faces remain in contact with the blade or cutting member 30, the interior of the tube parts can be kept sterile.

The cutting member 30 is then removed from between the tube parts rearranged without creating an open connection, while the severed ends/cut end faces of the tubes are surrounded by a curable material layer that is typically in a molten or softened state such that the two tube parts in alignment are fixed together, indirectly via the curable material M that ensures a welding connection J' (Fig. 6).
The first tube holder 41 and the second holder 42 are configured to be moved closer and axially, and preferably joined together, immediately after a removal of the cutting member 30 to allow a pair of new connections J' to be obtained at the two reconnection areas.

The device Bw is provided with the movable support 12 that supports one amongst the first holder 41 and the second holder 42, on a given same side relative to the cutting member 30, so that a rotation (180° rotation) can be performed: the new /rearranged alignment of the tube parts Ta with Tb, and Tb with Ta, respectively, is obtained. Then the material M adapted to form the bonding layer can be filled or released, for instance just after the removal of the cutting member 30 and after the axial contacting step 55.

Regarding the reconnection and sealing operation 500, it is understood the device Bw can allow the material M to fill the annular gap around the tube section Ts and around the tube region Tr, beneath the corresponding sleeve portions. After performing the axial contacting step 55 (with removal of the cutting member 30 along the separating plane P), such material filling allows material M to be present along the rearranged tube. The material M can be sufficiently flowable to entirely fill the respective annular gaps. A bonding layer thus can be formed continuously, across the separating plane P.

### Connection using a bonding material inside an annular collection zone

In some embodiments, the sleeves 3 are provided with an injection port 15 to allow the curable material M to be filled around the tubes at a gap area of the wrapped assembly, which an area forming a collection zone ZC after the inversion 54.
For instance, the injection via the injection port 15 is performed, while a pressure action (typically already exerted for the axial contacting step 55) is still maintained. The bonding material, curable, can be injected into the collection zone ZC (Fig. 6) that has been formed after the cut and the rearrangement. When forming end flanges by the respective sleeve end parts 3c, 3d, the collection zone ZC is closed on all sides. Possibly, the injection port can have a channel provided with a non-return valve V3, as schematically shown in Fig. 6 for instance.

When the material M consists of a silicone mass that extends annular in the collection zone ZC, around the central axis X3, the annular periphery around the tube contact established at the contacting step 55 is entirely filled with silicone that is molten (in a curable form). Of course, the silicone mass or similar material M can be already present in the collection zone ZC or poured in advance for a wrapped state, before the tube part A is pushed against the tube part B. The injection and the molding/forming step 56 start entirely after the contacting step 55, to allow forming a tube overlapping layer of the curable material M. Then, after a curing step 57, an axial pressure removal operation can be performed.

More generally, the curable material M can be injected or released to coat all or part of the pins or protrusions and surround the new contact area: the connection J' is obtained after curing this material M to define a layer of bonding material, not in contact with the interior/lumen of the tube parts Ta, Tb.
Thanks to the curing, an encapsulating layer is formed and the sleeves can become protecting sleeve that cover the encapsulating/sealing layer that is a permanent wrapping layer. A proper sealing of each tube connection at periphery of such connection, at least when recombined with the curable material being cured and forming a bonding layer filling the interior molding space of the sleeve plastic pieces forming the interlocked sleeve parts.

Referring to Fig. 9, a sleeve 3' is provided with curable material M already forming a layer along the inner face FM, with pins or protrusions 14 possibly being present to structurally reinforce/strengthen an interior face of the sleeve 3'. The sleeve 3' is squeezable to pinche the corresponding tube T or T'. A membrane 38 can cover the interior molding space MZ and the material M filling this interior molding space MZ can be in contact with the cutting member 30 after the cutting step 53. With similar operations, a continuous bonding layer of the material M can be formed, around the new tube junction with each of the tubes T, T' still wrapped by sleeve cut portions that belong to the sleeve 3'. The membrane 38 is possibly peelable (entirely removable) or with only a part thereof being removable.

While the injection port 15 can have an insertion channel that may run radially and/or axially in relation to the central axis X3, various options for insertion channel(s) may be available. For example, there may even be two, three, four, five or more insertion channels available. The mass of the material can follow the profile of the tube, even in squeezed state, due to flowability of the material before the curing. Then, after the curing step 57, the material M reinforces or strengthens locally the tube T or T' where this latter is externally covered.
Each rearranged tubing fluid connection is formed with an annular junction providing an inner surface of substantially constant inner diameter. The release of the squeezed state is deferred and can be a last step, occurring after the junction by the curing/curable material M. In some options, after the release, the sleeve parts may be withdrawn to have the rearranged tubes uncovered.

The kit K may be configured to have interlocking members that definitively lock the assembled state of the sleeve 3. After the inversion/rearrangement, at the end of the reconnection and sealing operation 500, four tube sections (possibly silicone section), remain wrapped thanks to the sleeves 3, 3' of the kit K. The sleeves 3 are typically not reusable and/or remain coupled to a tubing including a tube part Ta and a tube part Tb. A step of disposal for these parts can be performed, once operations for fluid circulation through the tubes have been finished.
The kit K and the associated tubes T, T' can be used in a pharmaceutical media transfer method, for instance in a pharmaceutical discharge method for supplying a pharmaceutical medium from a starting reservoir to a target reservoir. In principle, the connection method can also be used in other pharmaceutical, biological, medical technology or other scientific or industrial processes.

### Non-limiting exemplary use of the tubes that are equipped with the kit

Referring to Figs 3 and 11, it can be seen the kit with the sleeves 3 can be of interest in situations where the fluid circulation through flexible/collapsible tubes T, T', possibly including each silicone, must be stopped and a disconnection - reconnection operation is required.
A pair of tubes T, T' such as the one to which the invention applies consist of tubes that make each possible the circulation, the passage, and the communication of a fluid, such as a biopharmaceutical fluid and such a tube may be typically interposed between two- or more-pouches. Such a tube T, T', usually having a circular cross-section, is typically made of silicone or plastic material like TPE or PVC, with the list not being limiting. Flexibles tubes T, T' of the type Tuflux^{®} TPE or Tuflux^{®} SIL are exemplary tubes suitable to be cut, while being adapted for biopharmaceutical use.

Here in this non-limiting embodiment, prior to connecting bioprocess container 8 to cell culture device/apparatus 80, in one embodiment, flow through cell culture connection tube T' and bioprocess tube T may be stopped. For example, the system may include flow stop devices TC and TC' (clamps typically) that may be operated to prevent flow through the tubes.
The bioprocess container 8 can be suspended from a frame 8f. In this way, gravity can be utilized to allow cell culture medium L to flow out into cell culture device 80.
To attach cell culture connection tube T' to bioprocess tube T, these tubes are firstly surrounded, locally, by a sleeve 3 this assembled as shown in step 51 of Fig. 1. Each wrapped tube T, T' is then loaded into the biowelding device Bw or similar device that can provide suitable housings 20 arranged parallel to a longitudinal axis X. For example, in one embodiment, each tube holder is removable from the biowelding device Bw.

Bioprocess tube T and cell culture connection tube T' are then loaded into biowelding device or similar cutting and reconnecting device Bw. Each tube is securely placed within the corresponding housing 20 formed by the tube holders (for instance designed as the tube holders 41, 42 of Fig. 5D). More generally, any pair of tubes T, T' can be
As shown in Fig. 11, the end of each tube extends past each housing 20. When the biowelding device Bw is loaded, both tube holders 41, 42 can be closed as shown in FIG. 3 or coupled with clamp members of clamping parts CP1, CP2, CP3, CP4.

After the bioprocess tube T and cell culture connection tube T' are loaded and squeezed together with their sleeves 3, the blade (cutting member 30, Fig. 4) may be inserted into the biowelding device Bw. The blade may, for example, have any suitable shape, such as a plate-like rectangular shape.

The tubes T, T', which may be a bioprocess tube and a cell culture connection tube as in Fig. 11 or any suitable fluid conveying device in the biopharmaceutical field, can be made from thermoplastic, elastomers or silicone polymers, not necessarily being compatible with a heating since the connection are obtained peripherally/around the flexible material of theses tubes. For example, each tube T, T' can be made from silicone polymer. As used herein, polymer may also refer to homopolymers, copolymers, block copolymers, random copolymers, terpolymers, and the like. In one embodiment, the composition used to make the tube T, T' includes a plasticizer.

Construction with symmetrical halves or sleeve parts and possibly with a separating plane P that also forms a symmetry plane may be preferred. Accordingly, if fluid has to be swept from the cut end, a same sealing is performed on both sides of the cutting sites, provided the tube holders 41 and 42 have similar structure and operating mode for the squeezing. The tubes T, T' can be efficiently closed and cut into sections in a given region so as to prevent the circulation, the passage, and the communication of fluid on either side of this region and to be able to have two separate tube portions after a cut, each being thus closed and provisionally sealed.

The kit of two sealing/protecting sleeves 3 allows connecting biopharmaceutical flexible tube ends of two tubes in an encapsulated configuration, in a easy way, possibly with various devices that have clamp members adapted to hold the sleeves 3 properly. Each sleeve 3 can have optionally an indexation member provided peripherally, forming a guiding and positioning relief for instance. This may provide an indexation in rotation to ensure the best/suitable positioning of each wrapped tube using a holder assembly HA that is matching with such sleeve. In some options, the indexation member can reflect an outer diameter or other characteristic size of the sleeve 3, so that a range of sleeves can only be used with a given range of tube holders 41, 42 (this may prevents errors, should a sleeve being too small in size as compared to a tube and sleeve holder that is adapted for wrapping assemblies of greater size.

Of course, the invention is not limited to the embodiments described above and provided only as examples. It encompasses the various modifications, alternative forms, and other variants conceivable to a skilled person within the context of the invention, and in particular any combinations of the various modes of operation described above, which may be taken separately or in combination.

For instance, while the device Bw as illustrated (in Fig. 3 for instance) includes two holders 41, 42 and clamp members adapted to be distributed on either sides of a separating plane P where the cutting member 30 (Fig. 4) can extend, with use of a blade or similar cutting member 30 entering the interspace Sp, two blades may be provided in a variant, forming first and second blades (parallel blades) that pass between the clamps and/or between, the holders. In such option, the first blade and second blade are provided at first and second workstations on the device. A guillotine-like arrangement using one or more blades may be involved.
At least when the curable material M is silicone, the tubes T and T' can also include silicone material, which may consist of more than 60 percent by weight, more than 75 percent by weight, more than 90 percent by weight, more than 95 percent by weight, or even more than 98 percent by weight silicone.

In some variants, the sleeve parts 3a, 3b can be assembled by using external fixation means, not provided integral with the external wall SW. Any locking member can be provided to have the interior molding space MZ formed radially between the tube T, T' and the sleeve inner face Fm.

## Claims

1. An encapsulated connection kit (K) provided with two protecting sleeves (3; 3') for surrounding two flexible tubes (T, T') of a biopharmaceutical fluid pathway or assembly and allowing a rearrangement with a pair of tube connections involving four cut end faces obtained from the two flexible tubes made of plastic or silicone, after a simultaneous cut of the two flexible tubes (T, T') at tube sections (Ts, Tr) thereof, wherein the kit (K) comprises a first sleeve and a second sleeve forming the two sleeves (3; 3'), which are each of resiliently deformable plastic material, each of the first sleeve and the second sleeve being a sleeve comprising:
- a central axis (X3);
- an interior molding space (MZ) delimited by an inner face (Fm);
- at least one linking area (J3) that allows a full or partial disconnection, and reconnection, of two sleeve parts (3a, 3b) of the sleeve that provide two interior subspaces for molding, so that a side opening is available for tube insertion between the two subspaces;
- a plurality of pins or protrusions (14) that project radially inward from the inner face (Fm), with the plurality of pins or protrusion (14) being distributed circumferentially around the central axis (X3),
wherein each of the first sleeve and the second sleeve is a radially collapsible sleeve that is:
- configured to be pinched at two opposite sides to close a lumen of the corresponding flexible tube (T, T') passing therethrough, under radial pushing effect of the plurality of pins or protrusions (14),
- provided with the inner face (Fm), in undeformed configuration, which has a generally circular section so that each of the two sleeves (3; 3') has an inner radius (r3), as measured between the inner face (Fm) and the central axis (X3) in a undeformed sleeve configuration,
wherein the pins or protrusions (14) are shorter than the inner radius, preferably shorter than half the inner radius, the pins or protrusions (14) being configured to contact the tube (T, T') and maintain a radial space between the tube (T, T') and the inner face (Fm), so that each interior molding space (MZ) can extend around the tube (T, T') and a curable material (M) can coat the pins or protrusions (14) and encapsulate each junction area where two of the cut end faces are axially joined.

2. The encapsulated connection kit according to claim 1, wherein the interior molding space (MZ) is delimited by two opposite axial end parts (3c, 3d) formed as constrictions that inwardly project from the inner face (Fm).

3. The encapsulated connection kit according to claim 1 or 2, further comprising the curable material (M) that is preferably flowable and configured to cover each inner face (Fm) and be cured after the cut and the rearrangement.

4. The encapsulated connection according to claim 1, 2 or 3, further comprising the curable material (M) that is silicon.

5. The encapsulated connection kit according to any of the preceding claims, wherein the first sleeve and the second sleeve are two identical pieces, each having a longitudinal hinge (h1) and each provided with interlocking members (33, 34) at the linking area (J3),
and wherein said two sleeve parts (3a, 3b) are two halves that can be cut at a midsection (MS) thereof by a same blade or cutting member (30) involved for the cut of the two flexible tubes.

6. The encapsulated connection kit according to any of the claims 1-5, wherein the first sleeve and the second sleeve each include an injection port (15) for a filling of the curable material (M) through a sleeve external wall (SW).

7. The encapsulated connection kit according to any of the claims 1-5, further comprising an injection tool filled with the curable material, the injection tool being provided with an injection tip configured to pass through sleeve thickness or via an injection port (15).

8. The encapsulated connection kit according to any of the claims 1-5, wherein at least one amongst the first sleeve and the second sleeve is provided with a layer of the curable material (M) that is kept along the inner face (Fm) and preferably covered by at least one peelable membrane (38).

9. The encapsulated connection kit according to any one of the preceding claims, wherein in each of the first sleeve and the second sleeve, the two sleeve parts each comprise:
- inner grooves (G) that extend parallel to the central axis (X3).
and wherein each of the first sleeve and the second sleeve, made or consisting of a plastic material more rigid than silicon, is squeezable in a squeezed state such that each of the grooves (G) is closed or reduced in the squeezed state, while the pins or protrusions that include said plastic material are rigid and configured to keep their size and shape in the squeezed state.

10. The encapsulated connection kit according to claim 2 alone or combined with any one of the claims 3-9, wherein the two opposite axial end parts, formed as constrictions, each comprise teeth (36) that protrude radially inward,
and wherein respective interspaces are provided between the teeth (36) in undeformed configuration.

11. The encapsulated connection kit according to claim 9 and claim 10, wherein in each of the first sleeve and the second sleeve in undeformed configuration, the two sleeve parts (3a, 3b) each comprise:
- first interspaces regularly distributed at different angular sectors and forming part of a first subgroup (SG1) of the interspaces, which are provided in one of the two opposite axial end parts.
- second interspaces regularly distributed at different angular sectors and forming part of a second subgroup (SG2) of the interspaces, which are provided in the other one of the two opposite axial end parts;
- respective grooves of the inner grooves, each in alignment with an interspace of the first subgroup (SG1) and with an interspace of the second subgroup (SG2).

12. The encapsulated connection kit according to any one of the preceding claims, wherein the plurality of pins or protrusions (14) comprise one or several rows of at least nine pins or protrusions distributed circumferentially around the central axis (X3).

13. A cutting and connecting device (Bw) for cutting two tubes of plastic or silicone by simultaneously cutting a first flexible tube (T) at a tube section (Ts) thereof and a second flexible tube (T') at a tube region (Tr) thereof, and then establishing a pair of connections (J') by fixing a first tube end of the first flexible tube (T), obtained by the cutting, to a tube cut end of the second flexible tube (T') and also fixing a second tube end of the first flexible tube (T), obtained by the cutting, to another tube cut end of the second flexible tube (T'), so that a rearranged tubing fluid connection for a biopharmaceutical fluid is formed,
wherein the device (Bw), which comprises the kit of two protecting sleeves according to any one of the preceding claims to form a first sleeve surrounding the first tube (T) at the tube section (Ts) and a second sleeve surrounding the second tube (T') at the tube region (Tr), further comprises:
- a tube holder assembly (HA) having a first tube and sleeve holder (41) and a second tube and sleeve holder (42) that are adjacent along a separating plane (P) and are forming a pair of receiving housings (20) to have the first sleeve and the second sleeve maintained parallel to said separating plane (P) with an interspace (Sp) and each perpendicular to the separating plane;
- clamping parts (CP1, CP2, CP3, CP4) included in or coupled to the tube holder assembly (HA) and allowing each sleeve to be squeezed between one of the holders (41; 42) and a clamp member of the clamping parts (CP1, CP2, CP3, CP4);
- a cutting member (30) configured to pass between the first holder (41) and the second holder (42), along said plane (P), the cutting member (30) being movable to cut the first sleeve and the second sleeve when passing in the interspace (Sp);
- a driving unit allowing to reduce a given size of each receiving housing (20), along a given clamping direction by a clamping action using the clamping parts (CP1, CP2, CP3, CP4), preferably simultaneously, so that two fluid free areas (FFA) are obtained where the clamping parts (CP1, CP2, CP3, CP4) are in a clamping configuration against the first sleeve to squeeze the tube section (Ts) and against the second sleeve to squeeze the tube region (Tr); and
- a movable support that supports one amongst the first holder (41) and the second holder (42), on a given same side relative to the cutting member (30), the movable support being configured to rotate at an angle of 180°, on said given same side, to displace the first holder or the second holder correspondingly;
wherein a least one amongst the first holder (41) and the second holder (42) is displaced by the driving unit to allow the first holder and the second holder to be axially closer after a removal of the cutting member (30) and obtain said pair of connections (J'),
and wherein the resiliently deformable plastic material of each of the first sleeve and the second sleeve allows, upon releasing of the clamping configuration, to have:
- two rearranged sleeves of same cross-section as the first sleeve and the second sleeve with a circular section around the corresponding tube (T, T'); and
- the pins or protrusions (14) coated or being configured to be coated by the curable material (M).

14. The cutting and connecting device according to claim 13, wherein the first holder (41) comprises:
- first axial abutment means to prevent the first sleeve axially leaving the corresponding receiving housing (20) along a first direction so that a first axial retaining is exerted by the first axial abutment means on the first sleeve with the first flexible tube (T) that extends beyond the first axial abutment means,
- second axial abutment means to prevent the first sleeve axially leaving said corresponding receiving housing (20) along a second direction opposite from the first direction so that a second axial retaining is exerted by the second axial abutment means on the first sleeve with the first flexible tube (T) that extends beyond said second axial abutment means and beyond two opposite axial sides of the tube holder assembly (HA), and wherein the second holder (42) is configured rotatable relative to the first holder (41).

15. The cutting and connecting device according to claim 13 or 14, further comprising a welding unit configured to perform junction of the two tubes, which are preferably silicone tubes, by curing the curable material (M), by a heating or UV irradiation operation exerted though each of the sleeves, along a circumference around:
- the tube section (Ts); and
- the tube region (Tr).

16. A method of joining a first fluid conveying tube to a second fluid conveying tube equal in inner diameter and/or outer diameter to the first tube, while also joining a third fluid conveying tube to a fourth fluid conveying tube equal in inner diameter and/or outer diameter to the third tube, the method comprising:
* (a) providing a first continuous tube and a second continuous tube, each made of plastic or silicone, one of which initially includes the first tube and the third tube formed as two complementary segments of the first continuous tube, the second continuous tube including the second tube and the fourth tube formed as two complementary segments of the second continuous tube;
* (b) wrapping locally at a tube section (Ts) the first continuous tube (T), by a first sleeve, and also wrapping locally at a tube region (Tr) the second continuous tube (T'), by a second sleeve, the first sleeve and the second sleeve being the two sleeves of the kit according to any one of the claims 1-13;
* (c) squeezing, by a tube holder assembly (HA) provided with clamping means, the tube section (Ts) and the tube region (Tr) by clamping, simultaneously, the first sleeve and the second sleeve with the pins or protrusions (14) maintaining a spacing in each interior molding space (MZ) between the inner face (Fm) and the corresponding tube (T, T');
* (d) performing the joining after a cut through the two sleeves (3), with each of the tube section (Ts) and the tube region (Tr) simultaneously cut while the two pins or protrusions (14) of the sleeves maintain a squeezed state of the tube section (Ts) and the tube region (Tr).

17. The method according to claim 16, wherein the tube holder assembly (HA) and a cutting member (30) are used to transversely cut, along a cutting plane, the first and second continuous tubes, while the squeezed state of the tube section (Ts) and the tube region (Tr) is maintained by the tube holder assembly (HA) via a pressing contact transmitted via the pins or protrusions (14), the cutting along the cutting plane being performed to:
- separate the third fluid conveying tube from the first fluid conveying tube;
- separate the fourth fluid conveying tube from the second fluid conveying tube;
wherein the joining is performed, inside a pair of receiving housings of the tube holder assembly (HA) in an aseptic manner:
- after a relative rotation to have a first tube cut end in alignment with and facing a second tube cut end, along a direction perpendicular to the cutting plane to allow a first annular contact junction, and also a third tube cut end in alignment with and facing a fourth tube cut end, along a direction perpendicular to the cutting plane to allow a second annular contact junction; and
- after displacing the cutting member (30) away from a cutting site, by maintaining a squeezed state without any axial gap at the cutting site and immediately performing the first annular contact junction and the second annular contact junction, with the curable material provided to be cured in a collection zone (ZC) around respectively the first annular contact junction and the second annular contact junction.

18. The method according to claim 17, wherein the curable material (M) is provided under form of an inner layer along each inner face (Fm) of the respective sleeves (3), before the cut and rearrangement operation.
